# EUROPEAN PATENT APPLICATION

(11) **EP 1 253 144 A1**
(43) Date of publication of application: **30.10.2002**
(21) Application number: 01904317.3
(22) Date of filing: 02.02.2001
(51) Int. Cl.: C07D 271/10, C07D 413/06

(54) **1,3,4-OXADIAZOLE DERIVATIVES AND PROCESS FOR PRODUCING THE SAME**

(30) Priority: 03.02.2000 JP 2000026717
(71) Applicant: ONO PHARMACEUTICAL CO., LTD., Osaka-shi, Osaka 541-8526 (JP)
(72) Inventor: MIYAZAKI, Toru, Minase Research Institute, Osaka 618-8585 (JP); SUGIURA, Tsuneyuki, Minase Research Institute, Osaka Osaka 618-8585 (JP); HORIUCHI, Toshihide, Minase Research Institute, Osaka 618-8585 (JP)
(74) Representative: Henkel, Feiler, Hänzel
(86) International application number: JP0100741
(87) International publication number: WO01057004

(57) **Abstract**

A process for the preparation of compounds represented by formulae (I) and (IV): 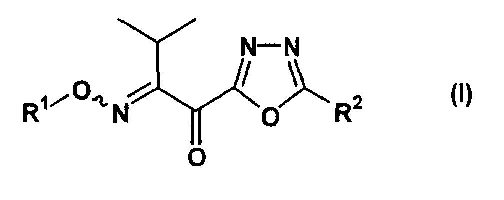 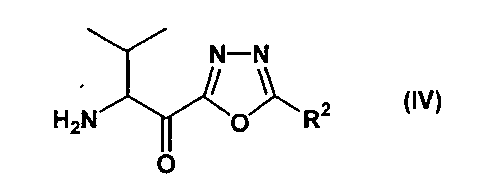 (wherein symbols in the formulae are described in the description) and the novel 1,3,4-oxadiazole derivative compound represented by formula (I). According to the present invention, preparation of intermediates (compounds of formula (IV)) of 1,3,4-oxadiazole derivatives useful as pharmaceuticals (elastase inhibitors) can be prepared efficiently via novel synthesis intermediates (compounds of formula (I)).

## Description

### TECHNICAL FIELD

The present invention relates to novel 1,3,4-oxadiazole derivatives and a process for the preparation thereof.

More particularly, the present invention relates to,
(1) a process for the preparation of compounds represented by formulae (I) and (IV): 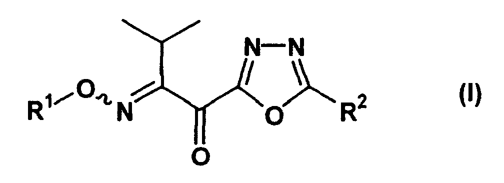 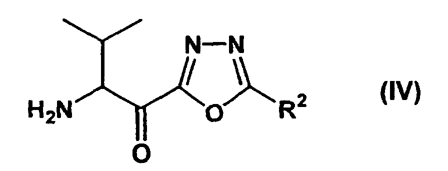 which are useful as synthesis intermediates of 1,3,4-oxadiazole derivatives useful as pharmaceuticals, and
(2) novel 1,3,4-oxadiazole derivatives represented by formula (I) obtained by the above preparation process.

### BACKGROUND OF THE INVENTION

As 1,3,4-oxadiazole derivatives useful as pharmaceuticals, for example, (1) Published Japanese Translation of PCT International Publication for Patent Application No. 10-511933 discloses that compounds represented by formulae (Z-a) to (Z-f): 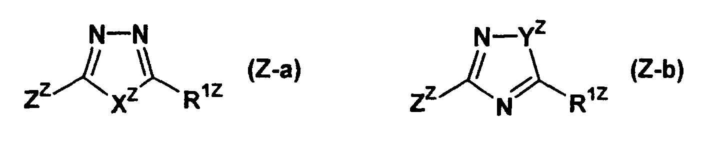 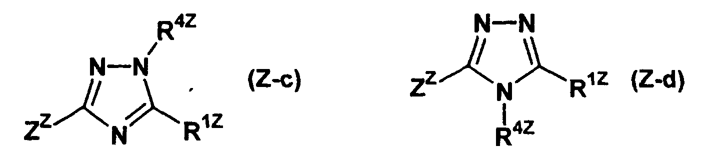 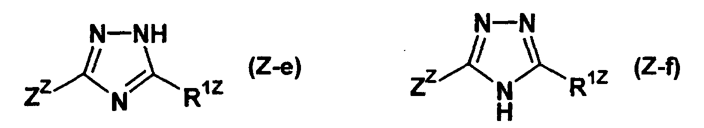 are useful as serine protease (particularly, elastase) inhibitors. However, the Preparation Methods or Examples of the specification describe only on 1,2,4-oxadiazole but not on 1,3,4-oxadiazole.
(2) WO 98/24806 discloses that compounds represented by formulae (W-a) to (W-c): 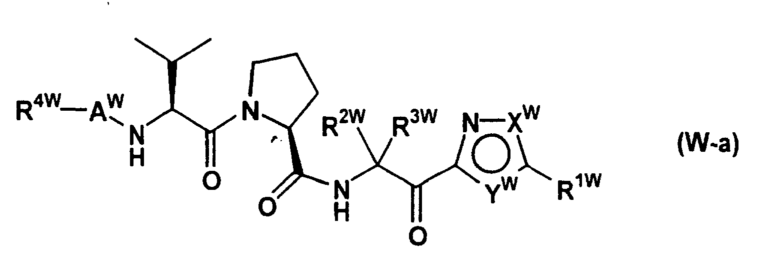 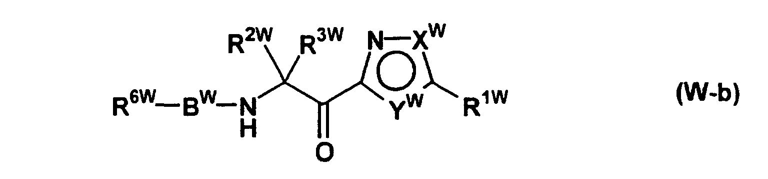 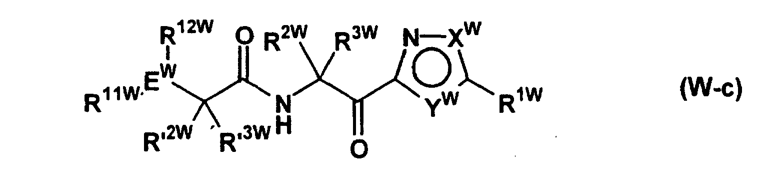 are useful as serine protease (particularly, elastase) inhibitors. In the specification, various compounds are synthesized via a compound represented by formula (W-1): 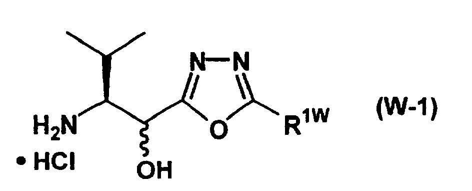 (in the formula, R^{1W} represents various substituting groups), and the compound is markedly important as an intermediate of pharmaceuticals.

Regarding the preparation process of the compound represented by formula (W-1), there is a description that it is prepared by the following reaction scheme 1 or 2.

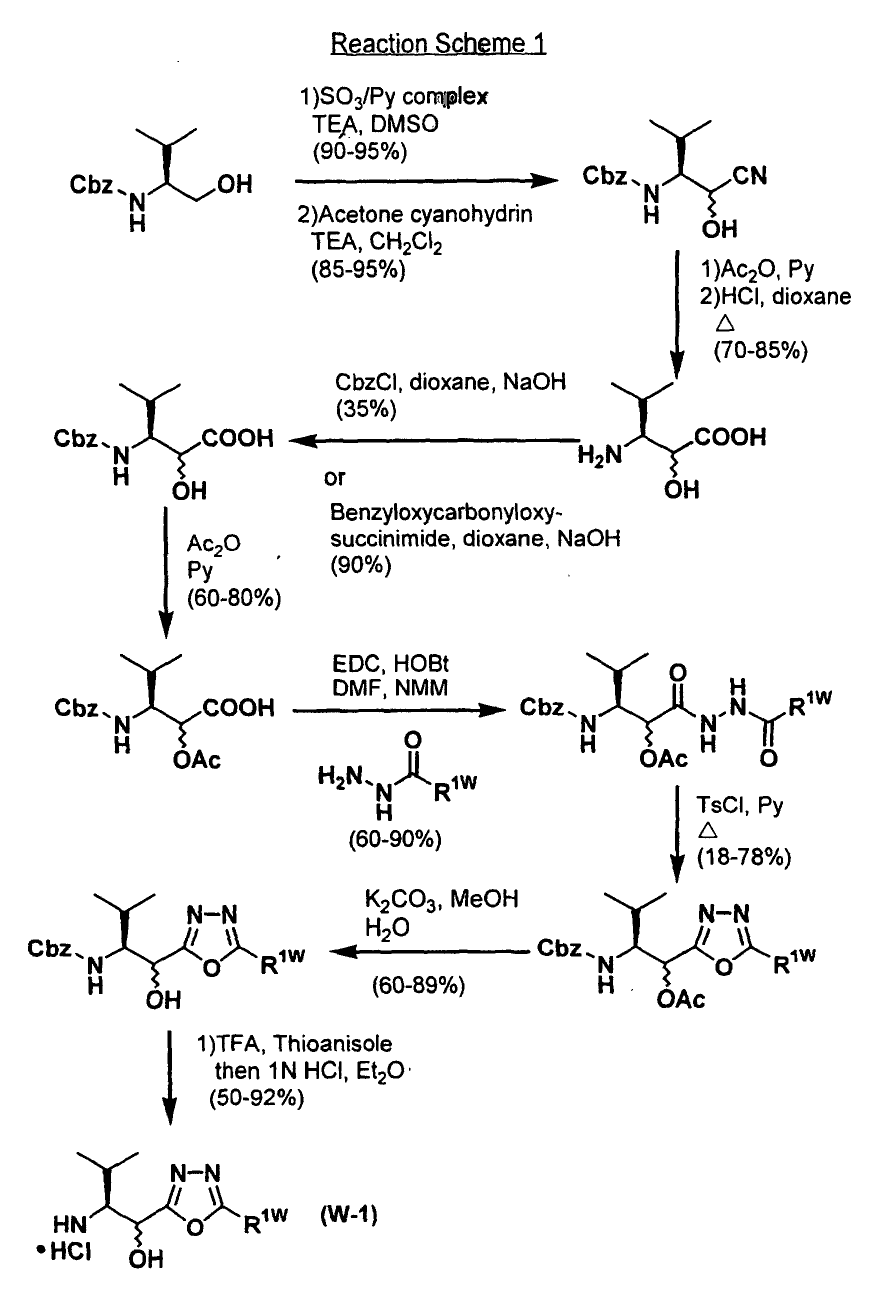

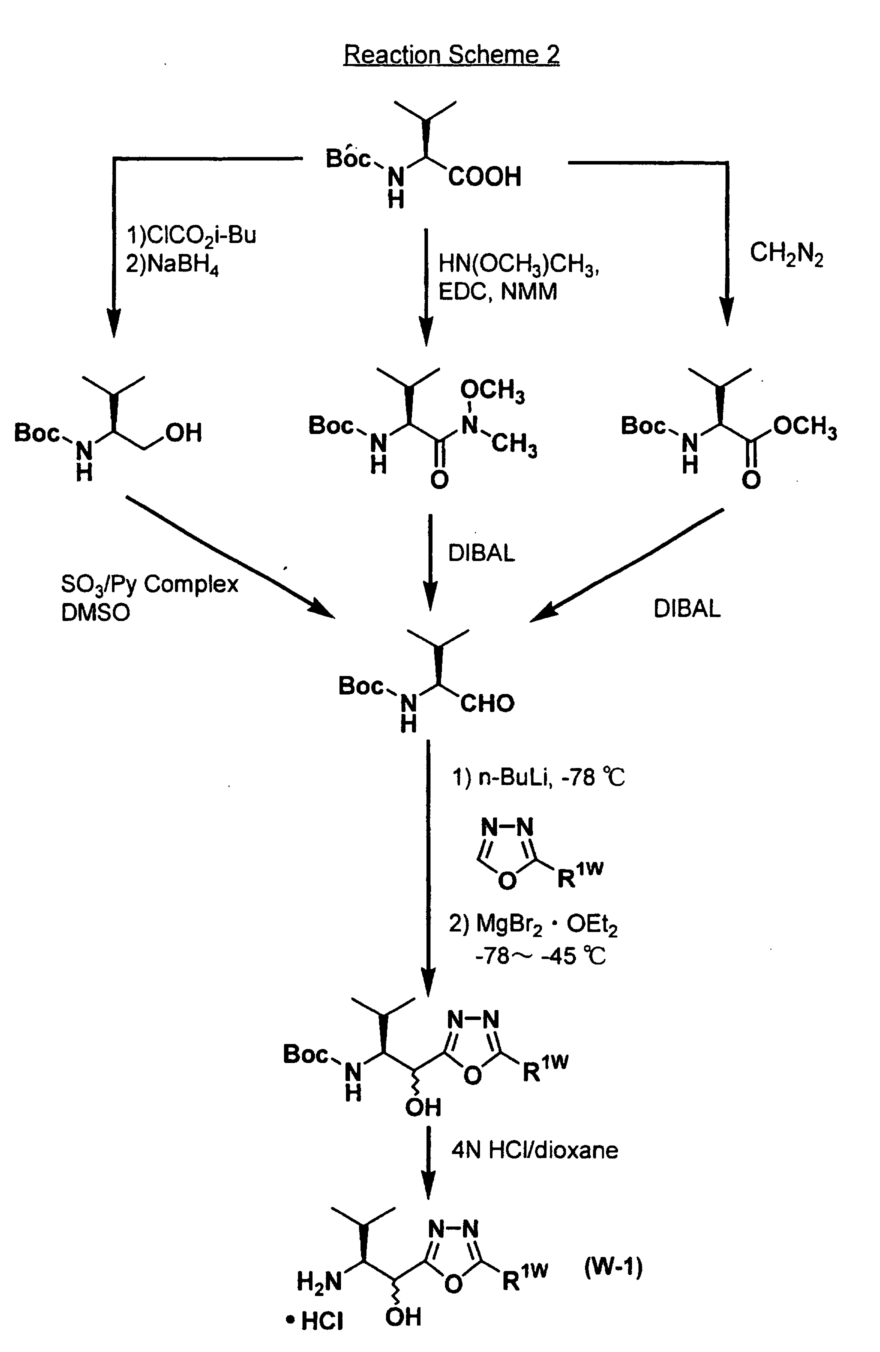

In the above reaction schemes,
R^{1W} represents various substituents,
Cbz represents a benzyloxycarbonyl group,
TEA represents triethylamine,
DMSO represents dimethyl sulfoxide,
Ac represents an acetyl group,
Py represents pyridine,
EDC represents 1-ethyl-3-[3-(dimethylamino)propyl]carbodiimide,
HOBt represents 1-hydroxybenzotriazole,
NMM represents N-methylmorpholine,
DMF represents dimethylformamide,
TsCI represents p-toluenesulfonyl chloride,
TFA represents trifluoroacetic acid,
Boc represents a t-butoxycarbonyl group, and
DIBAL represents diisobutylaluminum hydride.

(3) WO 00/55145 discloses that a novel compound represented by formula (Y): 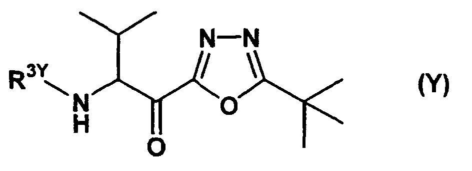 (in the formula, R^{3Y} represents a hydrogen atom or a protecting group of an amino group) and nontoxic salts thereof or hydrates thereof are useful as intermediates of pharmaceuticals. Regarding the preparation process, there is a description that it is prepared by the following reaction scheme 3.

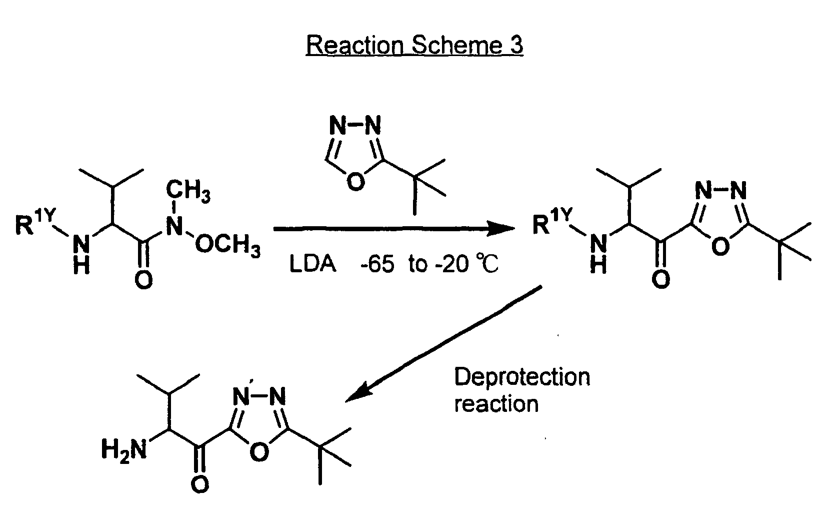

In the reaction scheme 3,
R^{1Y} represents a protecting group of an amino group, and
LDA represents lithium diisopropylamide.

In the conventional process shown by the reaction scheme 1, the compound represented by formula (W-1) is prepared from a material via a considerably large number of steps (10 steps), so that it was not sufficiently satisfactory as an industrial large scale synthesis method. Also, in the conventional processes shown by the reaction scheme 2 and reaction scheme 3, reactions at a low temperature (-78 to -20°C) are present so that they were not always satisfactory methods as industrial large scale synthesis methods. Accordingly, a process for the preparation of 1,3,4-oxadiazole derivatives which can be synthesized in an industrially large scale is required.

### DISCLOSURE OF THE INVENTION

Taking these problems involved in the background art into consideration, the present inventors have conducted intensive studies and found as a result of the efforts that a process shown by the following reaction scheme 4. 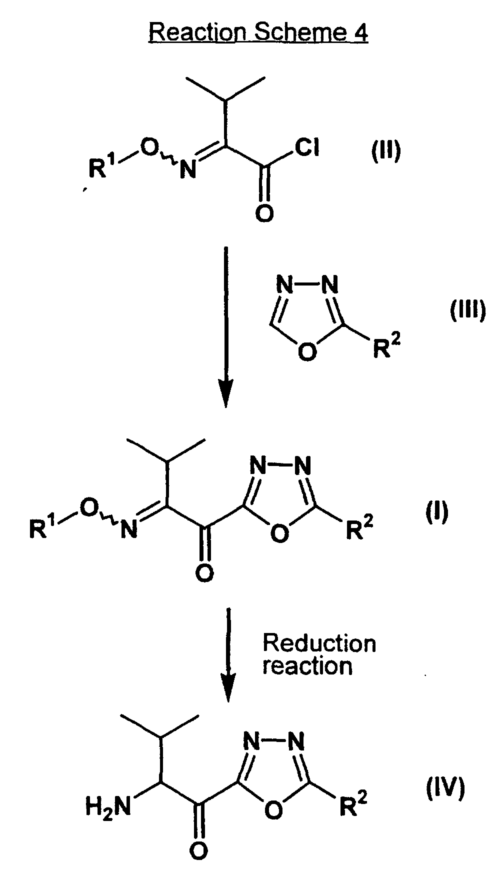 In the reaction scheme 4,

R¹ represents a C1-4 alkyl group or a C1-4 alkyl group substituted with a phenyl group, with the proviso that the phenyl group may be substituted with a C1-4 alkyl group, a C1-4 alkoxy group or a halogen atom, and

R² represents a phenyl group or 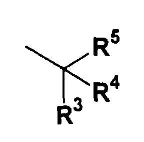 (wherein R³, R⁴ and R⁵ each independently represents (1) a hydrogen atom, (2) a C1-8 alkyl group, (3) a C3-7 cycloalkyl group, (4) a phenyl group, (5) a phenyl group substituted with 1 to 3 substituents selected from a C1-8 alkyl group, a C1-8 alkoxy group, a halogen atom, a trifluoromethyl group and a trifluoromethoxy group or (6) a 3,4-methylenedioxyphenyl group, or (7) R³ and R⁴ are taken together to represent a C2-6 alkylene group).

According to the process shown in the above reaction scheme 4 found by the present inventors, the compound represented by formula (IV) which is important as an intermediate of pharmaceuticals can be prepared by fewer steps than those of the conventional processes, without requiring a low temperature reaction.

Also, in the reaction scheme 4, the compound represented by formula (I) as an important intermediate of known serine protease (particularly elastase) inhibitors is a novel compound.

The reaction of an acid halide with a 5-membered heterocyclic ring is well known (cf., *Liebigs Ann. Chem*., 145-158 (1977), and *Liebigs Ann. Chem*., 159-168 (1977)). For example, regarding imidazole derivatives, the reaction shown by the following scheme (X-1): 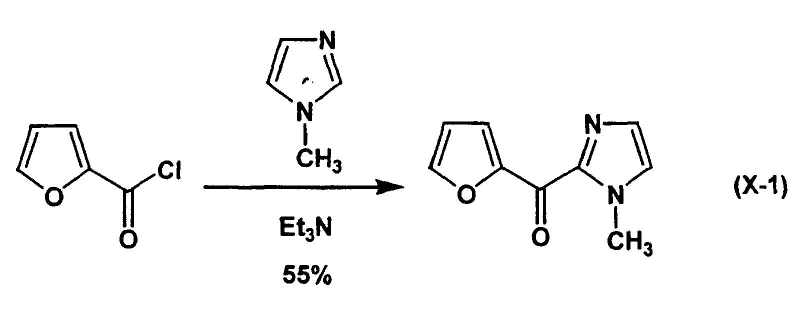 is known.

On the other hand, regarding 1,3,4-oxadiazole derivatives, the reaction shown by the following scheme (X-2): 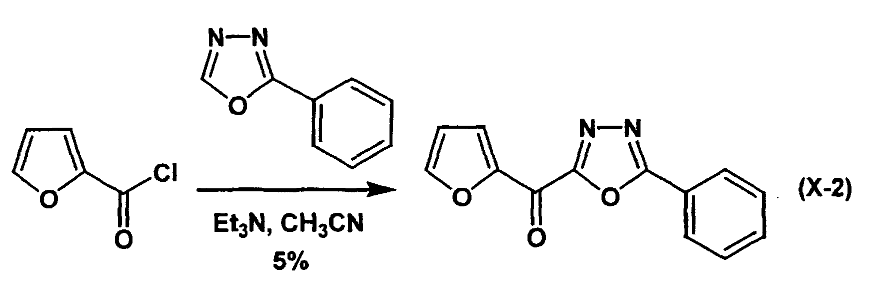 is known.

It is suggested from the references that achievement of high yield by the reaction of an acid halide with a 1,3,4-oxadiazole derivative is extremely difficult.

Actually, when 3-methyl-2-(methoxyimino)butyryl chloride and 2-(t-butyl)-1,3,4-oxadiazole were allowed to react in triethylamine by the process described in the references, 1-(5-(t-butyl)-1,3,4-oxadiazol-2-yl)-3-methyl-2-(methoxyimino)butan-1-one as the compound of interest was not able to obtain at a high yield.

According to the reaction of the present invention, the 1,3,4-oxadiazole derivative represented by formula (I) can be prepared efficiently while controlling formation of by-products, by allowing 1.0 equivalent of the compound represented by formula (II) to react with 1.0 equivalent of the 1,3,4-oxadiazole derivative represented by formula (III) at 0 to 20°C in the presence of a Lewis acid.

Accordingly, the present invention relates to
1) a process for the preparation of a 1,3,4-oxadiazole derivative compound represented by formula (I): 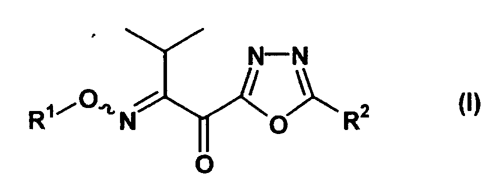 (symbols in the formula have the same meanings as described below), which comprises reacting a compound represented by formula (II): 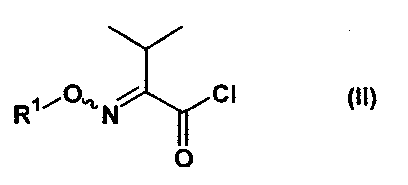 (wherein R¹ represents a C1-4 alkyl group or a C1-4 alkyl group substituted with a phenyl group, with the proviso that the phenyl group may be substituted with a C1-4 alkyl group, a C1-4 alkoxy group or a halogen atom) with a 1,3,4-oxadiazole derivative represented by formula (III): 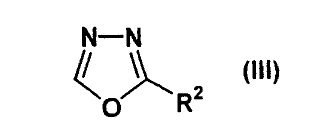 (wherein R² represents a phenyl group or 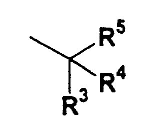 (wherein R³, R⁴ and R⁵ each independently represents (1) a hydrogen atom, (2) a C1-8 alkyl group, (3) a C3-7 cycloalkyl group, (4) a phenyl group, (5) a phenyl group substituted with 1 to 3 substituents selected from a C1-8 alkyl group, a C1-8 alkoxy group, a halogen atom, a trifluoromethyl group and a trifluoromethoxy group or (6) a 3,4-methylenedioxyphenyl group, or (7) R³ and R⁴ are taken together to represent a C2-6 alkylene group)),
2) a process for the preparation of a 1,3,4-oxadiazole derivative compound represented by formula (IV): 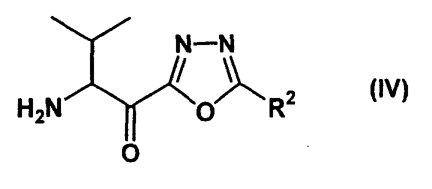 (wherein R² has the same meaning as described above) from a 1,3,4-oxadiazole derivative represented by a formula (I): 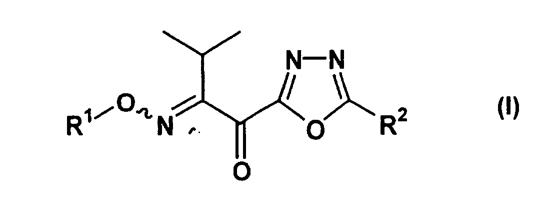 (symbols in the formula have the same meanings as described above), and
3) a novel 1,3,4-oxadiazole derivative compound represented by formula (I): 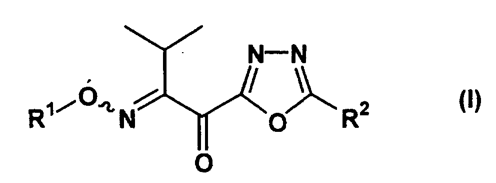 (wherein R¹ and R² have the same meanings as described above).

### DETAILED DESCRIPTION OF THE INVENTION

In formula (I), the C1-4 alkyl groups represented by R¹ and a substituent of the phenyl group of R¹ include methyl, ethyl, propyl and butyl groups and isomer groups thereof.

In formula (I), the C1-4 alkoxy groups represented by R¹ and a substituent of the phenyl group of R¹ include methoxy, ethoxy, propoxy and butoxy groups and isomer groups thereof.

In formula (I), the halogen atoms represented by a substituent of the phenyl group of R¹ and R³, R⁴ and R⁵ include fluorine atom, chlorine atom, bromine atom and iodine atom.

In formula (I), the C1-8 alkyl groups represented by R³, R⁴ and R⁵ include methyl, ethyl, propyl, butyl, pentyl, hexyl, heptyl and octyl groups and isomer groups thereof.

In formula (I), the C1-8 alkoxy groups represented by R³, R⁴ and R⁵ include methoxy, ethoxy, propoxy, butoxy, pentyloxy, hexyloxy, heptyloxy and octyloxy groups and isomer groups thereof.

In formula (I), the C3-7 cycloalkyl groups represented by R³, R⁴ and R⁵ include cyclopropyl, cyclobutyl, cyclopentyl, cyclohexyl and cycloheptyl groups and isomer groups thereof.

In formula (I), the C2-6 alkylene groups represented by taking R³ and R⁴ together include ethylene, trimethylene, tetramethylene, pentamethylene and hexamethylene groups and isomer groups thereof.

According to the present invention, unless otherwise indicated and as is apparent for those skilled in the art, each of symbols 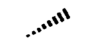 indicates that it is bound to the opposite side of the sheet (namely α-configuration), 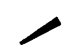 indicates that it is bound to the front side of the sheet (namely β-configuration), 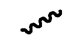 indicates that it is α-, β- or a mixture thereof, or E-isomer, Z-isomer or a mixture thereof, and 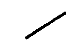 indicates that it is a mixture of α-configuration and β-configuration.

Unless otherwise indicated, all of these isomers are included in the present invention. For example, straight chains and branched chains are included in the alkyl groups, the alkoxy groups and the alkylene groups. In addition, all of isomers based on a double bond, a ring and a condensed ring (E-, Z-, cis- and trans-isomer), isomers due to the presence of an asymmetric carbon (R- and S-configuration, α- and β-configuration, enantiomers and diastereomers), optically active isomer having optical activity (D-, L-, d- and I-isomer), polar compounds obtained by chromatographic separation (more polar compounds and less polar compounds), equilibrium compounds, mixtures thereof at optional ratio and racemic mixtures are included in the present invention.

Among compounds of the present invention represented by formula (I), preferred examples include the following compounds:
(1) 1-(5-(t-butyl)-1,3,4-oxadiazol-2-yl)-3-methyl-2-(methoxyimino)butan-1-one,
(2) (E)-1-(5-(t-butyl)-1,3,4-oxadiazol-2-yl)-3-methyl-2-(methoxyimino)butan-1-one,
(3) (Z)-1-(5-(t-butyl)-1,3,4-oxadiazol-2-yl)-3-methyl-2-(methoxyimino)butan-1-one,
(4) 1-[5-(1-(benzo-1,3-dioxolen-5-yl)-1-methylethyl)-1,3,4-oxadiazol-2-yl]-3-methyl-2-(methoxyimino)butan-1-one,
(5) (E)-1-[5-(1-(benzo-1,3-dioxolen-5-yl)-1-methylethyl)-1,3,4-oxadiazol-2-yl]-3-methyl-2-(methoxyimino)butan-1-one,
(6) (Z)-1-[5-(1-(benzo-1,3-dioxolen-5-yl)-1-methylethyl)-1,3,4-oxadiazol-2-yl]-3-methyl-2-(methoxyimino)butan-1-one,
(7) 1-[5-phenyl-1,3,4-oxadiazol-2-yl]-3-methyl-2-(methoxyimino)butan-1-one,
(8) (E)-1-[5-phenyl-1,3,4-oxadiazol-2-yl]-3-methyl-2-(methoxyimino)butan-1-one,
(9) (Z)-1-[5-phenyl-1,3,4-oxadiazol-2-yl]-3-methyl-2-(methoxyimino)butan-1-one,
(10) 1-[5-(1-(1-methylethyl)-1,3,4-oxadiazol-2-yl]-3-methyl-2-(methoxyimino)butan-1-one,
(11) (E)-1-[5-(1-methylethyl)-1,3,4-oxadiazol-2-yl]-3-methyl-2-(methoxyimino)butan-1-one,
(12) (Z)-1-[5-(1-methylethyl)-1,3,4-oxadiazol-2-yl]-3-methyl-2-(methoxyimino)butan-1-one,
(13) 1-[5-(cyclopropylmethyl)-1,3,4-oxadiazol-2-yl]-3-methyl-2-(methoxyimino)butan-1-one,
(14) (E)-1-[5-(cyclopropylmethyl)-1,3,4-oxadiazol-2-yl]-3-methyl-2-(methoxyimino)butan-1-one,
(15) (Z)-1-[5-(cyclopropylmethyl)-1,3,4-oxadiazol-2-yl]-3-methyl-2-(methoxyimino)butan-1-one,
(16) 1-(5-(t-butyl)-1,3,4-oxadiazol-2-yl)-3-methyl-2-(benzyloxyimino)butan-1-one,
(17) (E)-1-(5-(t-butyl)-1,3,4-oxadiazol-2-yl)-3-methyl-2-(benzyloxyimino)butan-1-one, or
(18) (Z)-1-(5-(t-butyl)-1,3,4-oxadiazol-2-yl)-3-methyl-2-(benzyloxyimino)butan-1-one.

### Preparation process of the compound of the present invention:

The compound of the present invention represented by formula (I) can be prepared by the following process or the process described in Examples.

That is, the compound of the present invention represented by formula (I): 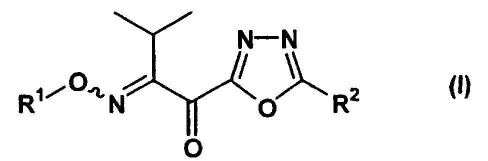 (wherein all of the symbols have the same meanings as described above) can be prepared by reacting a compound represented by formula (II): 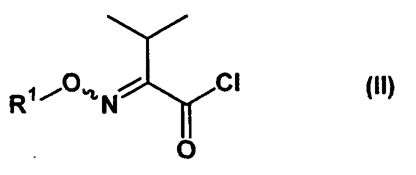 (wherein R¹ has the same meaning as described above) with a 1,3,4-oxadiazole derivative represented by formula (III): 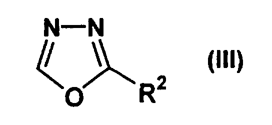 (wherein R² has the same meaning as described above).

The compound represented by formula (II) is allowed to react with the 1,3,4-oxadiazole derivative represented by formula (III), for example, by a reaction at a temperature of from 0 to 30°C in an organic solvent (acetonitrile, dimethylformamide, acetone, pyridine, or the like) in the presence of a Lewis acid (a mixture of trimethylsilyl triflate, trimethylsilyl iodide or trimethylsilyl chloride with an iodide (potassium iodide or sodium iodide, etc.), aluminum trichloride, titanium tetrachloride, iron trichloride, or the like) and a tertiary amine (N-methylmorpholine, dimethylaminopyridine, triethylamine, or the like).

The compound of the present invention represented by formula (I) can be optionally converted into the compound represented by formula (IV) which is important as an intermediate of parmaceuticals, or salts thereof, by the following process or the process described in Examples.

That is, the compound represented by formula (IV): 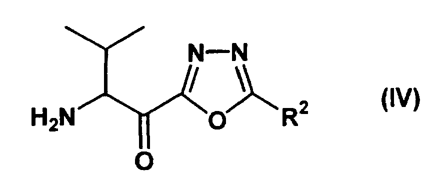 (wherein all symbols have the same meanings as described above) can be prepared by subjecting the compound of the present invention represented by formula (I) to a reduction reaction.

The reduction reaction is well known and can be carried out by a reduction reaction using a hydrolysis reaction.

The hydrolysis reaction is well known, and a deprotection reaction by the hydrolysis is carried out ,e.g., at a temperature of from -20 to 200°C in an inert solvent [an ether (e.g., tetrahydrofuran, dioxane, dimethoxyethane, diethyl ether, *etc.*)*,* an alcohol (e.g., methanol, ethanol, isopropyl alcohol, *etc*.), a benzene (e.g., benzene, toluene, *etc*), an amide (e.g., dimethylformamide, *etc.*)*,* water, ethyl acetate, acetic acid, a mixed solvent of two or more of them, or the like] in the presence of a hydrogenation catalyst (e.g., palladium-carbon, palladium black, palladium, palladium hydroxide, platinum-carbon, platinum dioxide, nickel, ruthenium chloride, *etc.*)*,* in the presence or absence of an inorganic acid (e.g., hydrogen chloride, sulfuric acid, hypochlorous acid, boric acid, tetrafluoroboric acid, *etc.*) or of an organic acid (e.g., acetic acid, p-toluenesulfonic acid, oxalic acid, trifluoroacetic acid, formic acid, *etc.*) or in the presence or absence of trimethylsilyl chloride, trimethylsilyl bromide or trimethylsilyl iodide in hydrogen atmosphere under ordinary or compressed conditions or in the presence of ammonium formate. When an acid is used, salt thereof may be used.

The compound represented by formula (I) as the starting material of the reduction reaction is converted into the compound represented by formula (IV), even when it is E-isomer, Z-isomer or an E/Z mixture.

The compound represented by formula (II) can be prepared in accordance with the process of the following reaction scheme 5.

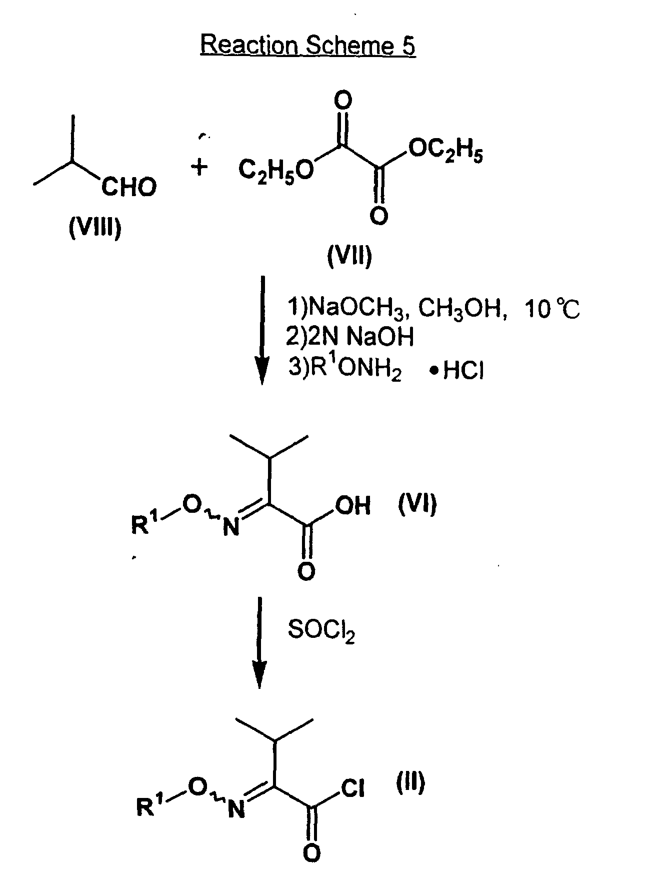

The compound represented by formula (III) can be prepared in accordance with the process of the following reaction scheme 6.

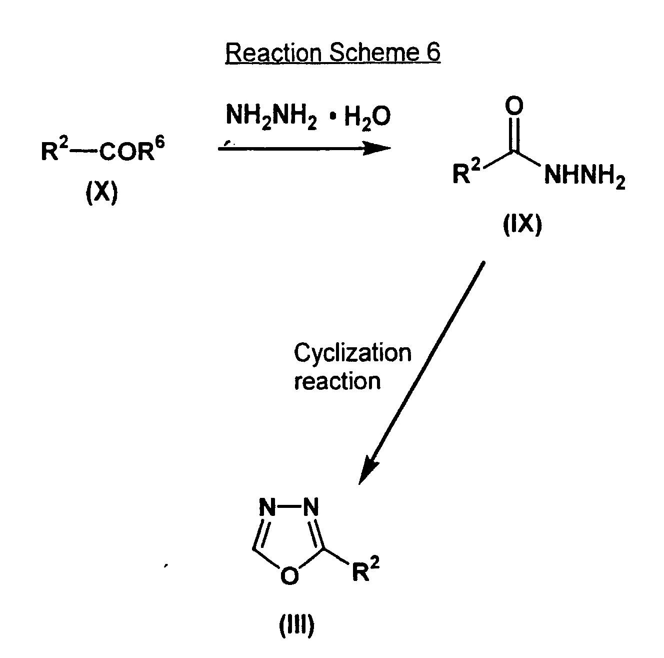

In the above reaction schemes, R⁶ represents a hydroxyl group, a halogen atom or a C1-4 alkoxy group, and other symbols have the same meanings as described above.

A product of each reaction may be subjected to the subsequent reaction by carrying out isolation, washing, drying and purification at each step or without carrying out these operations, or subjected to the subsequent step by suspending it after appropriate operations. The reaction product by each reaction can be purified by conventional techniques such as distillation at atmospheric or reduced pressure, high performance liquid chromatography, thin layer chromatography or column chromatography using silica gel or magnesium silicate, washing and recrystallization.

The compound represented by formula (IV) can be converted into acid addition products and hydrates thereof by known methods.

Examples of the salt as used herein include salts of alkali metals (potassium, sodium, *etc.*), salts of alkaline earth metals (calcium, magnesium, *etc*.), ammonium salts, salts of pharmacologically acceptably organic amines (tetramethylammonium, triethylamine, methylamine, dimethylamine, cyclopentylamine, benzylamine, phenetylamine, piperidine, monoethanolamine, diethanolamine, tris(hydroxymethyl)aminomethane, lysine, arginine and N-methyl-D-glucamine, *etc.*) and acid addition salts (inorganic acid salts such as hydrochloride, hydrobromide, sulfate, phosphate and nitrate, or organic acid salts such as acetate, trifluoroacetate, lactate, tartarate, oxalate, fumarate, maleate, citrate, benzoate, methanesulfonate, ethanesulfonate, benzenesulfonate, toluenesulfonate, isethionate, glucuronate, gluconate, etc.). These salts can be prepared by known methods.

In addition, the compounds described herein or salts thereof can also be converted into hydrates by known methods.

### BEST MODE FOR CARRYING OUT THE INVENTION

The present invention is described below in detail based on reference examples and examples, but the present invention is not limited thereto.

In the chromatography separation and TLC, the solvents shown in parentheses show eluting or developing solvents, and their ratio is volume ratio. The solvent shown in parentheses in NMR is a solvent used in the measurement.

### Reference Example 1

### 3-Methyl-2-(methoxyimino)butyric acid:

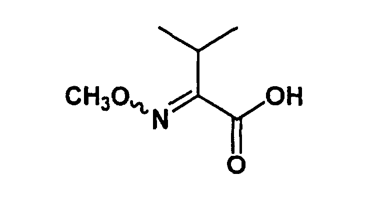

Under an argon, diethyl oxalate (400 mL) was gradually added dropwise to a methanol solution of 28% sodium methylate (568 g) under cooling with ice. The reaction mixture was stirred at the same temperature for 1 hour. Isobutyl aldehyde (300 mL) was gradually added dropwise to the reaction mixture. The reaction mixture was stirred at room temperature for 30 minutes. The reaction mixture was again under cooling with ice , and 2 N aqueous sodium hydroxide solution (1.77 L) was gradually added thereto. The reaction suspension was stirred at 20°C for 1 hour. O-Methyl-hydroxylamine hydrochloride (319 g) was added several times to the thus obtained reaction suspension. The reaction mixture was stirred at room temperature for 14 hours. The thus obtained reaction suspension was filtered, and the residue was washed with methanol. The filtrates (4.5 L) concentrated to evaporate about 2.5 L of the solvent. A 5 N aqueous sodium hydroxide solution (550 mL) was gradually added to the remaining suspension. The resulting solution was extracted twice with t-butyl methyl ether (500 mL), and concentrated hydrochloric acid (350 mL) was added to the thus obtained water layer under cooling with ice. The solution was extracted twice with ethyl acetate (1.5 L). The extracts were combined, dried over anhydrous magnesium sulfate and then concentrated to obtain the title compound (301 g) having the following physical properties.
TLC: Rf 0.45 (E-isomer, chloroform : methanol : acetic acid = 50 : 5 : 1) and 0.24 (Z-isomer, chloroform : methanol : acetic acid = 50 : 5 : 1)
NMR (CDCl₃): δ 4.04 and 4.00 (s each, 3H), 3.40 and 2.90 (m each, 1H), 1.23 and 1.18 (d each, J = 7.0 Hz, 6H)

### Reference Example 2

### 3-Methyl-2-(methoxyimino)butyryl chloride:

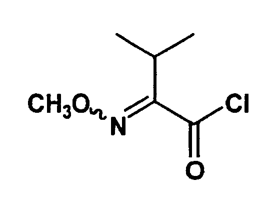

Thionyl chloride (500 mL) and dimethylformamide (0.2 mL) were added to the compound prepared in Reference Example 1 (301 g). The reaction mixture was refluxed for 1.5 hours. By distilling the reaction mixture under a reduced pressure, the title compound (262 g) having the following physical properties was obtained.
b.p.: 60°C/22 mmHg
NMR (CDCl₃): δ 4.14 and 3.91 (s each, 3H), 3.42 and 2.80 (m each, 1H), 1.22 and 1.18 (d each, J = 7.0 Hz, 6H)

### Reference Example 3

### 2,2-Dimethylpropionyl hydrazide:

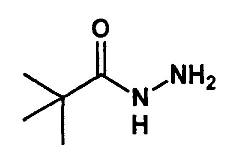

Under an argon, a mixture of methyl pivalate (1,040 mL) and hydrazine hydrate (760 mL) was refluxed at 120°C for 15 hours. The reaction mixture was cooled to room temperature and then concentrated. The thus obtained residue was stirred under cooling with ice , and the thus formed solid was filtered and washed with hexane to obtain the title compound (500 g) having the following physical properties. In addition, the filtrate was concentrated and again stirred under cooling with ice , and the thus formed solid was filtered and washed with hexane to obtain the title compound (72 g) having the following physical properties.
TLC: Rf 0.59 (chloroform : methanol = 10 : 1)
NMR (CDCl₃): δ 7.08 (brs, 1H), 3.89 (brs, 2H), 1.21 (s, 9H)

### Reference Example 4

### 2-(t-butyl)-1,3,4-Oxadiazole:

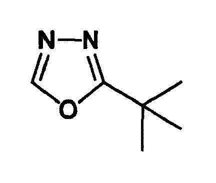

A mixture of the compound prepared in Reference Example 3 (570 g), methyl orthoformate (805 mL) and p-toluenesulfonic acid monohydrate (14 g) was stirred under heating to evaporate methanol, followed by stirring until methanol distillation ceased. The reaction mixture was cooled to room temperature and distilled under a reduced pressure to obtain the title compound (538 g) having the following physical properties.
b.p.: 80°C/18 mmHg
TLC: Rf 0.31 (hexane : ethyl acetate = 2 : 1)
NMR (CDCl₃): δ 8.33 (s, 1H), 1.44 (s, 9H)

### Reference Examples 4(1) to 4(4)

By the same procedure as a series of reaction of Reference Example 3 → Reference Example 4 using a corresponding compound instead of methyl pivalate, the following title compounds were obtained.

### Reference Example 4(1)

### 2-(1-(Benzo-1,3-dioxolen-5-yl)-1-methylethyl)-1,3,4-oxadiazole:

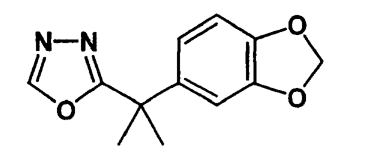

TLC: Rf 0.36 (hexane : ethyl acetate = 1 : 1)
NMR (CDCl₃): δ 8.30 (s, 1H), 6.82-6.72 (m, 3H), 5.94 (s, 2H), 1.81 (s, 6H)

### Reference Example 4(2)

### 2-Phenyl-1,3,4-oxadiazole:

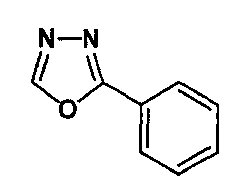

b.p.: 95°C/3 mmHg
TLC: Rf 0.32 (hexane : ethyl acetate = 2 : 1)
NMR (CDCl₃): δ 8.48 (s, 1H), 8.12-8.06 (m, 2H), 7.61-7.40 (m, 3H)

### Reference Example 4(3)

### 2-(1 -Methylethyl)-1,3,4-oxadiazole:

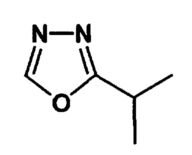

b.p.: 74°C/25 mmHg
TLC: Rf 0.32 (hexane : ethyl acetate = 2 : 1)
NMR (CDCl₃): δ 8.35 (s, 1H), 3.24 (m, 1H), 1.42 (d, J = 7.0 Hz, 6H)

### Reference Example 4(4)

### 2-(1-Methylcyclopropyl)-1,3,4-oxadiazole:

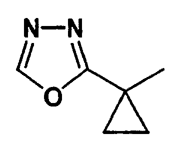

b.p.: 92°C/17 mmHg
TLC: Rf 0.28 (hexane : ethyl acetate = 2 : 1)
NMR (CDCl₃): δ 8.23 (s, 1H), 1.57 (s, 3H), 1.30 (m, 2H), 0.96 (m, 2H)

### Example 1

### (E)-1-(5-(t-Butyl)-1,3,4-oxadiazol-2-yl)-3-methyl-2-(methoxyimino)butan-1-one:

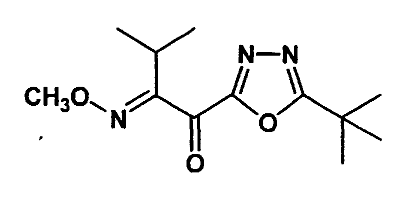

Under an argon and, trimethylsilyl chloride (0.25 mL), N-methylmorpholine (3.3 mL) and the compound prepared in Reference Example 4 (1.28 g) were added successively to a solution of acetonitrile (10 mL) and pyridine (2.4 mL) containing sodium iodide (300 mg) under cooling with ice. The compound prepared in Reference Example 2 (1.65 g) was gradually added to the reaction mixture under cooling with ice . The reaction mixture was stirred at room temperature for 20 hours. The reaction mixture was diluted with ethyl acetate, washed with 1 N hydrochloric acid, a saturated aqueous sodium bicarbonate solution and a saturated aqueous sodium chloride solution in this order, dried over anhydrous magnesium sulfate and then concentrated. By purifying the thus obtained residue by silica gel column chromatography (hexane : ethyl acetate = 10:1), a compound of the present invention (2.18 g) having the following physical properties was obtained.
TLC: Rf 0.50 (hexane : ethyl acetate = 3 : 1)
NMR (CDCl₃): δ 4.09 (s, 3H), 3.45 (m, 1H), 1.48 (s, 9H), 1.26 (d, J = 7.0 Hz, 6H)

### Examples 1(1) to 1(4)

By the same procedure as a series of reaction of Example 1 using the compound prepared in Reference Example 4(1) to 4(4) instead of the compound prepared in Reference Example 4, the following compounds of the present invention were obtained.

### Example 1(1)

### (E)-1-[5-(1-(Benzo-1,3-dioxolen-5-yl)-1-methylethyl)-1,3,4-oxadiazol-2-yl]-3-methyl-2-(methoxyimino)butan-1-one:

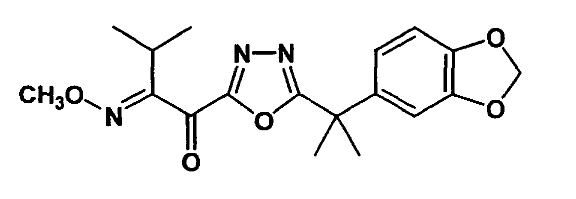

TLC: Rf 0.38 (hexane : ethyl acetate = 2 : 1)
NMR (CDCl₃): δ 6.82-6.70 (m, 3H), 5.94 (s, 2H), 4.04 (s, 3H), 3.41 (m, 1H), 1.83 (s, 6H), 1.23 (d, J = 7.2 Hz, 6H)

### Example 1(2)

### (E)-1-[5-Phenyl-1,3,4-oxadiazol-2-yl]-3-methyl-2-(methoxyimino)butan-1-one:

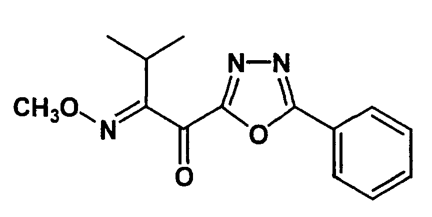

TLC: Rf 0.38 (hexane : ethyl acetate = 2 : 1)
NMR (CDCl₃): δ 8.22-8.13 (m, 2H), 7.64-7.49 (m, 3H), 4.12 (s, 3H), 3.48 (m, 1H), 1.29 (d, J = 6.9 Hz, 6H)

### Example 1(3)

### (E)-1-[5-(1-Methylethyl)-1,3,4-oxadiazol-2-yl]-3-methyl-2-(methoxyimino)butan-1-one:

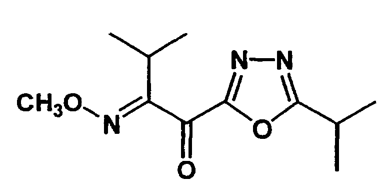

TLC: Rf 0.49 (hexane : ethyl acetate = 2 : 1)
NMR (CDCl₃): δ 4.09 (s, 3H), 3.44 (m, 1H), 3.28 (m, 1H), 1.45 (d, J = 7.2 Hz, 6H), 1.26 (d, J = 7.2 Hz, 6H)

### Example 1(4)

### (E)-1-[5-(Cyclopropylmethyl)-1,3,4-oxadiazol-2-yl]-3-methyl-2-(methoxyimino)butan-1-one:

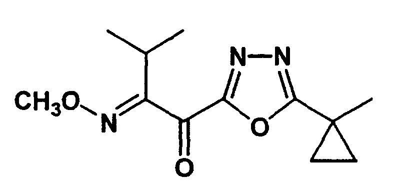

TLC: Rf 0.52 (hexane : ethyl acetate = 2 : 1 )
NMR (CDCl₃): δ 4.08 (s, 3H), 3.43 (m, 1H), 1.61 (s, 3H), 1.41 (m, 2H), 1.24 (d, J = 7.0 Hz, 6H), 1.03 (m, 2H)

### Example 2

### (1RS)-1-(5-(t-Butyl)-1,3,4-oxadiazol-2-ylcarbonyl)-2-methylpropylamine hydrochloride:

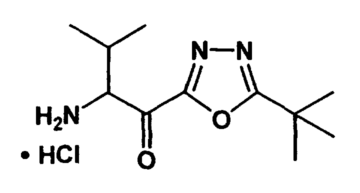

To an isopropyl alcohol (17 mL) solution of the compound prepared in Example 1 (1.021 g), 5% platinum-carbon (0.2 g) and a 4 N hydrogen chloride-dioxane solution (3.0 mL) were added. The reaction mixture was stirred for 2 hours under hydrogen at 5 atmospheric pressure under cooling with ice. Using a glass filter, the reaction mixture was filtered and washed with isopropyl alcohol. The filtrate was mixed with toluene and concentrated, and the thus obtained residue was mixed with toluene and concentrated. The thus obtained residue was recrystallized from ethyl acetate and t-butyl methyl ether, filtered and then dried to obtain a compound of the present invention (563 mg) having the following physical properties.
TLC: Rf 0.49 (chloroform : methanol = 10 : 1)
NMR (DMSO-d₆): δ 8.81 (brs, 3H), 4.71 (d, J = 4.6 Hz, 1H), 2.49 (m, 1H), 1.40 (s, 9H), 1.08 (d, J = 4.4 Hz, 3H), 0.92 (d, J = 4.4 Hz, 3H)

### Example 2(1)

### (1 RS)-1 -(5-(t-Butyl)-1,3,4-oxadiazol-2-ylcarbonyl)-2-methylpropylamine hydrochloride:

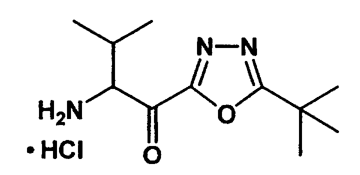

5% platinum-carbon (0.2 g) and trimethylsilyl chloride (6.1 mL) were added to isopropyl alcohol (20 mL) solution of the compound prepared in Example 1 (4.05 g) under cooling with ice. The reaction mixture was stirred for 3 hours under hydrogen at atmospheric pressure under cooling with ice. Using a glass filter, the reaction mixture was filtered and washed with isopropyl alcohol. The filtrate was concentrated, and the thus obtained residue was mixed with toluene and concentrated. The thus obtained residue was recrystallized from ethyl acetate and t-butyl methyl ether, filtered and then dried to obtain the compound of the present invention (3.5 g) having the same physical properties of Example 2.

### Example 2(2)

### (1RS)-1-(5-(t-Butyl)-1,3,4-oxadiazol-2-ylcarbonyl)-2-methylpropylamine hydrochloride:

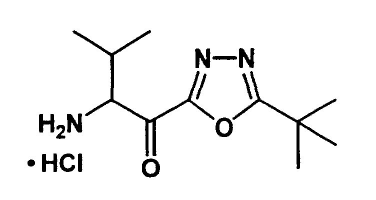

5% platinum-carbon (0.1 g) and trimethylsilyl chloride (3 mL) were added to an ethanol (13 mL) solution of the compound prepared in Example 1 (4.05 g) under cooling with ice. The reaction mixture was stirred for 5 hours under hydrogen at atmospheric pressure under cooling with ice. Using a glass filter, the reaction mixture was filtered and washed with ethanol. The filtrate was concentrated, and the thus obtained residue was mixed with toluene and concentrated. The thus obtained residue was recrystallized from ethyl acetate and t-butyl methyl ether, filtered and then dried to obtain the compound of the present invention (3.33 g) having the same physical properties of Example 2.

### Example 2(3)

### (1 RS)-1-(5-(t-Butyl)-1,3,4-oxadiazol-2-ylcarbonyl)-2-methylpropylamine hydrochloride:

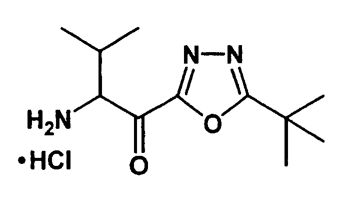

5% platinum-carbon (0.2 g) and a 3.90 N hydrogen chloride-ethanol solution (6.2 mL) were added to ethanol (13 mL) solution of the compound prepared in Example 1 (4.05 g) under cooling with ice. The reaction mixture was stirred for 2 hours under hydrogen at atmospheric pressure under cooling with ice. Using a glass filter, the reaction mixture was filtered and washed with ethanol. The filtrate was concentrated, and the thus obtained residue was mixed with toluene and concentrated. The thus obtained residue was recrystallized from ethyl acetate and t-butyl methyl ether, filtered and then dried to obtain the compound of the present invention (3.08 g) having the same physical properties of Example 2.

### Example 3(1) or 3(2)

By the same procedure as a series of reaction of Example 2 using the compound prepared in Example 1(1) or Example 1(4) instead of the compound prepared in Example 1, the following compounds of the present invention were obtained.

### Example 3(1)

### (1RS)-1-[5-(1-(Benzo-1,3-dioxolen-5-yl)-1-methylethyl)-1,3,4-oxadiazol-2-ylcarbonyl]-2-methylpropylamine hydrochloride:

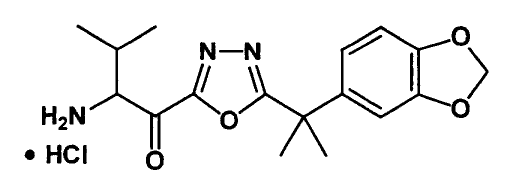

TLC: Rf 0.61 (chloroform : methanol = 10 : 1 )
NMR (DMSO-d₆): δ 8.76 (brs, 3H), 6.95-6.70 (m, 3H), 5.99 (s, 2H), 4.70 (d, J = 4.8 Hz, 1H), 1.76 (s, 6H), 1.04 (d, J = 6.9 Hz, 3H), 0.91 (d, J = 6.9 Hz, 3H)

### Example 3(2)

### (1 RS)-1-[5-(Cyclopropylmethyl)-1,3,4-oxadiazol-2-ylcarbonyl]-2-methylpropylamine hydrochloride:

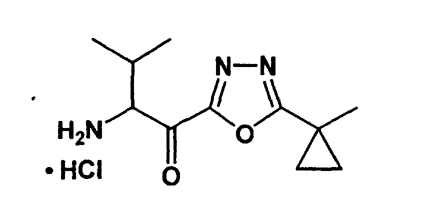

TLC: Rf 0.55 (chloroform : methanol = 10 : 1)
NMR (DMSO-d₆): δ 8.81 (brs, 3H), 4.70 (brs, 1H), 1.53 (s, 3H), 1.32 (m, 2H), 1.14 (m, 2H), 1.03 (d, J = 7.2 Hz, 3H), 0.90 (d, J = 7.2 Hz, 3H)

### Example 4(1) or 4(2)

By the same procedure as a series of reaction of Example 2(1) using the compound prepared in Example 1(2) or Example 1(3) instead of the compound prepared in Example 1, the following compounds of the present invention were obtained.

### Example 4(1)

### (1RS)-1-[5-Phenyl-1,3,4-oxadiazol-2-ylcarbonyl]-2-methylpropylamine hydrochloride:

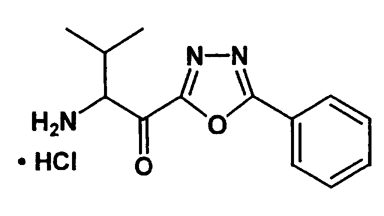

TLC: Rf 0.50 (chloroform : methanol = 10 : 1)
NMR (DMSO-d₆): δ 8.95-8.70 (brs, 3H), 8.13 (d, J = 8.1 Hz, 2H), 7.76-7.60 (m, 3H), 4.82 (d, J = 4.5 Hz, 1H), 2.55 (m, 1H), 1.09 (d, J = 6.9 Hz, 3H), 0.96 (d, J= 7.2 Hz, 3H)

### Example 4(2)

### (1 RS)-1-[5-(1-Methylethyl)-1,3,4-oxadiazol-2-ylcarbonyl)-2-methylpropylamine hydrochloride:

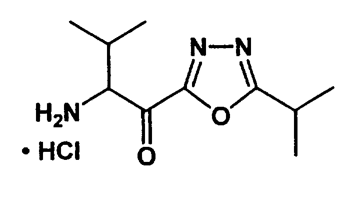

TLC: Rf 0.48 (chloroform : methanol = 10 : 1 )
NMR (DMSO-d₆): δ 8.90-8.70 (brs, 3H), 4.72 (d, J = 4.5 Hz, 1H), 3.34 (m, 1H), 2.49 (m, 1H), 1.35 (d, J = 6.9 Hz, 6H), 1.05 (d, J = 6.6 Hz, 3H), 0.92 (d, J = 6.9 Hz, 3H)

### Examples 5(1) and 5(2)

### (E)-1-(5-(t-Butyl)-1,3,4-oxadiazol-2-yl)-3-methyl-2-(methoxyimino)butan-1-one:

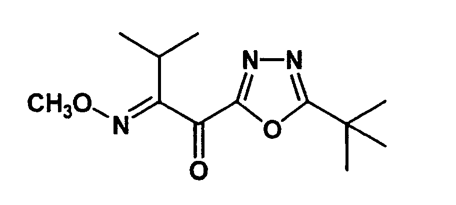

### (Z)-1 -(5-(t-Butyl)-1,3,4-oxadiazol-2-yl)-3-methyl-2-(methoxyimino)butan-1 -one:

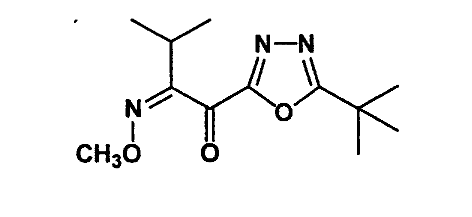

Under an argon, the compound prepared in Reference Example 2 (164 mg) was gradually added dropwise to an acetonitrile (1 mL) solution of the compound prepared in Reference Example 4 (126 mg) and N-methylmorpholine (0.11 mL) under cooling with ice. The reaction mixture was stirred at room temperature for 20 hours. The reaction mixture was diluted with ethyl acetate, washed with 1 N hydrochloric acid, a saturated aqueous sodium bicarbonate solution and a saturated aqueous sodium chloride solution in this order, dried over anhydrous magnesium sulfate and then concentrated. The thus obtained residue was purified by silica gel column chromatography (hexane : ethyl acetate = 10:1) to obtain E-isomer (52 mg) and Z-isomer (14 mg) of a compound of the present invention having the following physical properties.

### Example 5 (1): E-isomer

TLC: Rf 0.50 (hexane : ethyl acetate = 3 : 1)
NMR (CDCl₃): δ 4.09 (s, 3H), 3.45 (m, 1H), 1.48 (s, 9H), 1.26 (d, J = 7.0 Hz, 6H)

### Example 5 (2): Z-isomer

TLC: Rf 0.55 (hexane : ethyl acetate = 3 : 1)
NMR (CDCl₃): δ 3.79 (s, 3H), 2.88 (m, 1H), 1.49 (s, 9H), 1.23 (d, J = 7.0 Hz, 6H)

### Example 6

### (E)-1-(5-(t-Butyl)-1,3,4-oxadiazol-2-yl)-3-methyl-2-(methoxyimino)butan-1-one:

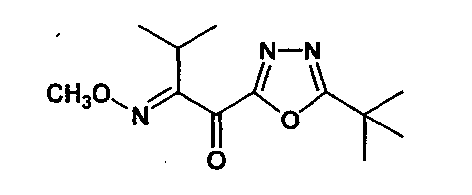

Under an argon and at 0°C or less, pyridine (299.3 g), trimethylsilyl chloride (32 mL), N-methylmorpholine (382.7 g) and the compound prepared in Reference Example 4 (159.1 g) were added in this order to an acetonitrile (1.26 L) solution of sodium iodide (37.8 g). The compound prepared in Reference Example 2 (206.3 g) was gradually added dropwise to the reaction mixture at 3°C. The reaction mixture was stirred overnight at 0°C. The reaction mixture was poured into 2 N hydrochloric acid (1.25 L) and extracted with ethyl acetate (2 L). The organic layer was washed with a 10% aqueous sodium hydrogen sulfite solution (1.25 L), and the water layer was extracted twice with ethyl acetate (600 mL). The organic layers were combined and washed with a saturated aqueous sodium bicarbonate solution (2 L), water (2 L) and a saturated aqueous sodium chloride solution (1.25 L) in this order. The mixture was stirred for 1 hour by adding activated carbon and anhydrous magnesium sulfate and then filtered, and the filtrate was concentrated. The thus obtained residue was recrystallized from hexane to obtain the compound of the present invention (230.2 g) having the following physical properties.
m.p.: 55.8-56.2°C
TLC: Rf 0.50 (hexane : ethyl acetate = 3 : 1)
NMR (CDCl₃): δ 4.09 (s, 3H), 3.45 (m, 1H), 1.48 (s, 9H), 1.26 (d, J = 7.0 Hz, 6H)

### Reference Example 5

### 3-Methyl-2-(benzyloxyimino)butyric acid chloride:

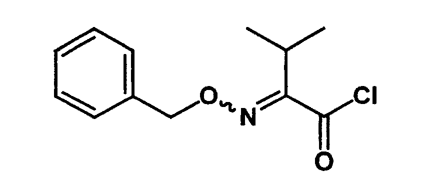

By the same procedure as a series of reaction of Reference Example 1 → Reference Example 2 using O-benzyl-hydroxylamine hydrochloride instead of O-methyl-hydroxylamine hydrochloride, the following title compound having the following physical properties was obtained.
NMR (CDCl₃): δ 7.5-7.2 (m, 5H), 5.34 (s, 2H), 3.43 (m, 1H), 1.17 (d, J = 7.0 Hz, 6H)

### Example 7

### (E)-1-(5-(t-Butyl)-1,3,4-oxadiazol-2-yl)-3-methyl-2-(benzyloxyimino)butan-1-one:

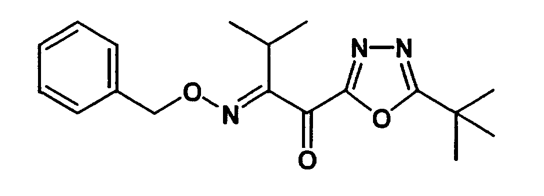

Under an argon, trimethylsilyl chloride (2.2 mL), N-methylmorpholine (28 mL) and the compound prepared in Reference Example 4 (10.5 g) were added in this order to a solution of acetonitrile (80 mL) and pyridine (21 mL) containing sodium iodide (2.5 g) under cooling with ice. The compound prepared in Reference Example 5 (26 g) was gradually added dropwise to the reaction mixture under cooling with ice. The reaction mixture was stirred at room temperature for 17 hours. The reaction mixture was diluted with ethyl acetate, washed with 2 N hydrochloric acid, a saturated aqueous sodium thiosulfate solution, a saturated aqueous sodium bicarbonate solution, water and a saturated aqueous sodium chloride solution in this order, dried over anhydrous magnesium sulfate and then concentrated. The thus obtained residue was purified by silica gel column chromatography (hexane : ethyl acetate = 20:1) to obtain the compound of the present invention (26.3 g) having the following physical properties.
TLC: Rf 0.47 (hexane : ethyl acetate = 2 : 1)
NMR (CDCl₃): δ 7.37 (m, 5H), 5.31 (s, 2H), 3.48 (m, 1H), 1.46 (s, 9H), 1.26 (d, J = 7.0 Hz, 6H)

### Example 8

### (1RS)-1-(5-(t-Butyl)-1,3,4-oxadiazol-2-ylcarbonyl)-2-methylpropylamine hydrochloride:

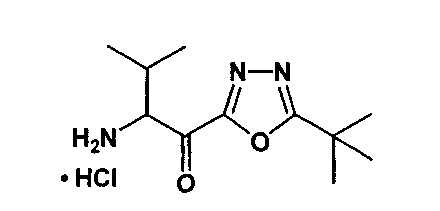

To an isopropyl alcohol (8.5 mL) solution of the compound prepared in Example 7 (668 mg), 5% platinum-carbon (100 mg) and a 4.3 N hydrogen chloride-isopropyl alcohol solution (1.5 mL) were added. The reaction mixture was stirred for 3 hours under hydrogen at 5 atmospheric pressure under cooling with ice. Using a glass filter, the reaction mixture was filtered and washed with isopropyl alcohol. The filtrate was mixed with toluene and concentrated, and the thus obtained residue was mixed with toluene and concentrated. The thus obtained residue was recrystallized from ethyl acetate and t-butyl methyl ether, filtered and then dried to obtain the compound of the present invention (226 mg) having the same physical properties of Example 2.

### INDUSTRIAL APPLICABILITY

According to the present invention, preparation of intermediates (compounds of formula (IV)) of 1,3,4-oxadiazole derivatives useful as pharmaceuticals (elastase inhibitors) can be prepared efficiently via novel synthesis intermediates (compounds of formula (I)), without requiring low temperature reactions such as the conventional processes and by smaller number of steps.

## Claims

1. A process for a preparation of a 1,3,4-oxadiazole derivative compound represented by formula (I): 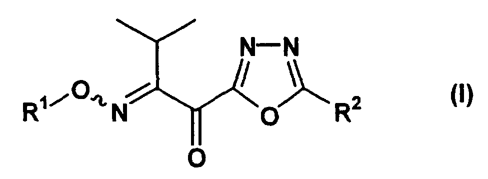 wherein symbols in the formula have the same meanings as described below,
which comprises:
reacting a compound represented by formula (II): 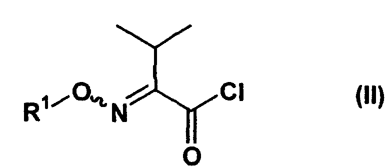 wherein R¹ represents a C1-4 alkyl group or a C1-4 alkyl group substituted with a phenyl group, in which the phenyl group may be substituted with a C1-4 alkyl group, a C1-4 alkoxy group or a halogen atom,
with a 1,3,4-oxadiazole derivative represented by formula (III): 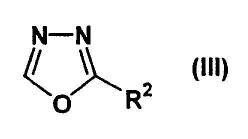 wherein R² represents a phenyl group or 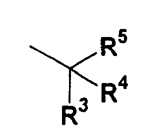 wherein R³, R⁴ and R⁵ each independently represents (1) a hydrogen atom, (2) a C1-8 alkyl group, (3) a C3-7 cycloalkyl group, (4) a phenyl group, (5) a phenyl group substituted with 1 to 3 substituents selected from a C1-8 alkyl group, a C1-8 alkoxy group, a halogen atom, a trifluoromethyl group and a trifluoromethoxy group or (6) a 3,4-methylenedioxyphenyl group, or (7) R³ and R⁴ are taken together to represent a C2-6 alkylene group.

2. A process for a preparation of a 1,3,4-oxadiazole derivative compound represented by formula (IV) or a salt thereof: 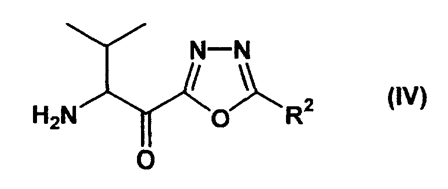 wherein R² has the same meaning as described below,
which comprises:
reacting a compound represented by formula (II): 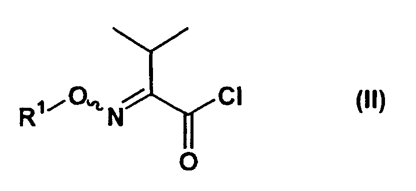 wherein R¹ has the same meaning as described in claim 1,
with a 1,3,4-oxadiazole derivative represented by formula (III): 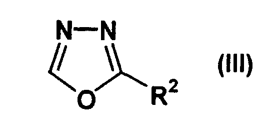 wherein R² has the same meaning as described in claim 1, to obtain a 1,3,4-oxadiazole derivative represented by a formula (I): 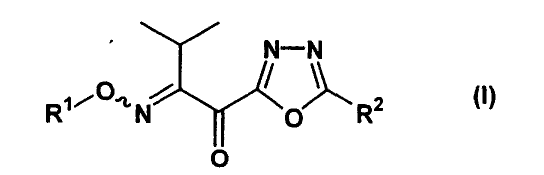 wherein symbols in the formula have the same meanings as described above, and
subjecting the 1,3,4-oxadiazole derivative to a reduction reaction, optionally followed by conversion to a salt.

3. A process for a preparation of a 1,3,4-oxadiazole derivative compound represented by formula (IV) or a salt thereof: 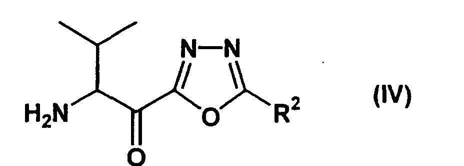 wherein R² has the same meaning as described below,
which comprises:
subjecting a 1,3,4-oxadiazole derivative represented by a formula (I): 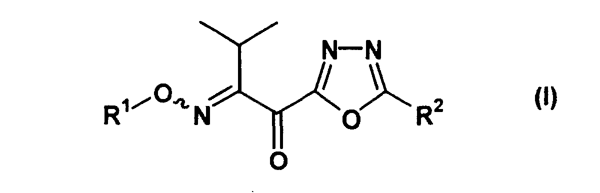 wherein symbols in the formula have the same meanings as described in claim 1,
to a reduction reaction, optionally followed by conversion to a salt.

4. A 1,3,4-oxadiazole derivative compound represented by formula (I): 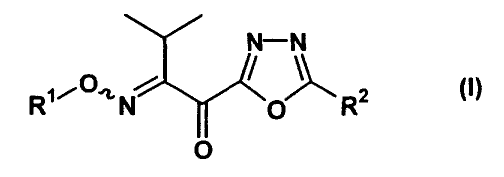 wherein R¹ and R² have the same meanings as described in claim 1.

5. The 1,3,4-oxadiazole derivative compound according to claim 4, which is
(1) 1-(5-(t-butyl)-1,3,4-oxadiazol-2-yl)-3-methyl-2-(methoxyimino)butan-1-one,
(2) (E)-1-(5-(t-butyl)-1,3,4-oxadiazol-2-yl)-3-methyl-2-(methoxyimino)butan-1-one,
(3) (Z)-1-(5-(t-butyl)-1,3,4-oxadiazol-2-yl)-3-methyl-2-(methoxyimino)butan-1-one,
(4) 1-[5-(1-(benzo-1,3-dioxolen-5-yl)-1-methylethyl)-1,3,4-oxadiazol-2-yl]-3-methyl-2-(methoxyimino)butan-1-one,
(5) (E)-1-[5-(1-(benzo-1,3-dioxolen-5-yl)-1-methylethyl)-1,3,4-oxadiazol-2-yl]-3-methyl-2-(methoxyimino)butan-1-one,
(6) (Z)-1-[5-(1-(benzo-1,3-dioxolen-5-yl)-1-methylethyl)-1,3,4-oxadiazol-2-yl]-3-methyl-2-(methoxyimino)butan-1-one,
(7) 1-[5-phenyl-1,3,4-oxadiazol-2-yl]-3-methyl-2-(methoxyimino)butan-1-one,
(8) (E)-1-[5-phenyl-1,3,4-oxadiazol-2-yl]-3-methyl-2-(methoxyimino)butan-1-one,
(9) (Z)-1-[5-phenyl-1,3,4-oxadiazol-2-yl]-3-methyl-2-(methoxyimino)butan-1-one,
(10) 1-[5-(1-(1-methylethyl)-1,3,4-oxadiazol-2-yl]-3-methyl-2-(methoxyimino)butan-1-one,
(11) (E)-1-[5-(1-methylethyl)-1,3,4-oxadiazol-2-yl]-3-methyl-2-(methoxyimino)butan-1-one,
(12) (Z)-1-[5-(1-methylethyl)-1,3,4-oxadiazol-2-yl]-3-methyl-2-(methoxyimino)butan-1-one,
(13) 1-[5-(cyclopropylmethyl)-1,3,4-oxadiazol-2-yl]-3-methyl-2-(methoxyimino)butan-1-one,
(14) (E)-1-[5-(cyclopropylmethyl)-1,3,4-oxadiazol-2-yl]-3-methyl-2-(methoxyimino)butan-1-one,
(15) (Z)-1-[5-(cyclopropylmethyl)-1,3,4-oxadiazol-2-yl]-3-methyl-2-(methoxyimino)butan-1-one,
(16) 1-(5-(t-butyl)-1,3,4-oxadiazol-2-yl)-3-methyl-2-(benzyloxyimino)butan-1-one,
(17) (E)-1-(5-(t-butyl)-1,3,4-oxadiazol-2-yl)-3-methyl-2-(benzyloxyimino)butan-1-one, or
(18) (Z)-1-(5-(t-butyl)-1,3,4-oxadiazol-2-yl)-3-methyl-2-(benzyloxyimino)butan-1-one.
